(19) Europäisches Patentamt
European Patent Office
Office européen des brevets

(11) EP 2 206 493 B1

(12) FASCICULE DE BREVET EUROPEEN

(45) Date de publication et mention de la délivrance du brevet:
**28.09.2011 Bulletin 2011/39**

(51) Int Cl.:
***A61K 8/68*** *(2006.01)* ***A61K 8/99*** *(2006.01)*
***A61Q 19/08*** *(2006.01)*

(21) Numéro de dépôt: **10150451.2**

(22) Date de dépôt: **11.01.2010**

# (54) Association cosmétique d'un microorganisme et d'un dérivé phytosphingosine

Kosmetische Kombination aus einem Mikroorganismen und einem Phytosphingosinderivat

Cosmetic combination of a microorganism and a phytosphingosine derivative

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **12.01.2009 FR 0950143**
**22.01.2009 US 146319 P**

(43) Date de publication de la demande:
**14.07.2010 Bulletin 2010/28**

(73) Titulaire: **L'Oréal**
**75008 Paris (FR)**

(72) Inventeurs:
- **Amar, David**
**75015, Paris (FR)**
- **Bernard, Bruno**
**92400, Courbevoie (FR)**
- **Bernard, Dominique**
**92170, Vanves (FR)**
- **Castiel, Isabelle**
**06200, Nice (FR)**

(74) Mandataire: **Le Coupanec, Pascale A.M.P.**
**Nony & Associés**
**3, rue de Penthièvre**
**75008 Paris (FR)**

(56) Documents cités:
**EP-A- 0 919 226 EP-A- 1 593 382**
**FR-A- 2 905 856**

- **PARAGH,SCHLING,UGOSCAI,LIEBISCH: "Novel sphingolipid derivatives promote keratinocyte differenciation" EXPERIMENTAL DERMATOLOGY, vol. 17, no. 12, 17 mars 2008 (2008-03-17) , pages 1004-1016, XP002543996**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention vise à proposer une association particulièrement avantageuse pour prévenir et/ou traiter le vieillissement cutané.

L'épiderme est un épithélium, conventionnellement divisé en une couche basale de kératinocytes contenant, notamment, des cellules souches cutanées et constituant la couche germinative de l'épiderme, une couche dite épineuse constituée de plusieurs couches de cellules polyhédriques disposées sur la couche basale, une couche dite granuleuse comprenant une à trois couches dites de cellules aplaties contenant des inclusions cytoplasmiques distinctes, les grains de kératohyaline, et enfin, un ensemble de couches supérieures, appelé couche cornée (ou *stratum corneum*) constituée de kératinocytes au stade terminal de leur différenciation appelés cornéocytes.

Le stratum corneum, ou couche cornée, est la couche superficielle de l'épiderme située à l'interface entre l'organisme et son environnement. Il est composé de cornéocytes, cellules anucléées résultant de la différenciation des kératinocytes épidermiques. Les cornéocytes sont riches en kératines et sont entourés d'une matrice - lipidique imperméable. De par sa composition en protéines et en lipides, le stratum corneum joue un rôle essentiel de barrière cutanée. Il empêche l'intrusion d'agents microbiologiques et permet de préserver l'hydratation de la peau et donc du corps en général.

Lors du vieillissement de la peau et en dehors des conséquences bien connues de l'âge sur le relief de la peau, de nombreux inconforts sont rapportés par les personnes âgées. Ces inconforts trouvent leur origine dans une altération de la fonction barrière et de l'homéostasie épidermique de leur peau. Ainsi, le stratum corneum des peaux âgées a une teneur en lipides intercellulaires décrue par rapport aux peaux jeunes, particulièrement pendant la période hivernale. Ce changement de composition du stratum corneum en perturbe ses propriétés physico-chimiques de barrière cutanée. Enfin, la vitesse de récupération de la fonction barrière après altération du stratum corneum est ralentie avec l'âge laissant supposer un dysfonctionnement de la fonction homéostasique de l'épiderme (Denda, M., 2002 ; Ghadially, R. et al, 1995 ; Leveque, J.L., 2001). La société Déposante a en outre constaté, dans ces mêmes circonstances, qu'en réponse à une agression physique ou chimique du stratum corneum, des gènes particuliers montrent une cinétique de modulation significativement différente selon l'âge du sujet. Plus précisément l'induction de l'expression de certains gènes s'avère être ralentie dans la peau âgée, en comparaison à la peau jeune

Parmi ces gènes dont l'expression est particulièrement ralentie chez les sujets âgés exposés à un stress, figure notamment le gène de la kératine 6B (KRT6B) qui a par ailleurs été caractérisé comme particulièrement intéressant au regard de son implication dans les processus de réparation et de régénération épidermique.

Par ailleurs, l'homéostasie de la peau, et en particulier de l'épiderme, résulte d'une balance finement régulée entre les processus de prolifération et de différenciation des cellules de la peau. Ces processus de prolifération et de différenciation sont parfaitement régulés : ils participent au renouvellement et/ou à la régénération de la peau et conduisent au maintien d'une épaisseur constante de la peau, et en particulier d'une épaisseur constante de l'épiderme. Cette homéostasie de la peau participe également au maintien des propriétés mécaniques de la peau.

Mais cette homéostasie de la peau peut être altérée par certains facteurs physiologiques (âge, ménopause, hormones...), ou environnementaux (stress UV, pollution, stress oxydant, stress irritant...). Le potentiel régénératif de l'épiderme devient moins important : les cellules de la couche basale se divisent moins activement, conduisant notamment à un ralentissement et/ou une diminution du renouvellement épidermique. Par conséquent, le renouvellement cellulaire ne compense plus la perte des cellules éliminées en surface, conduisant à une atrophie de l'épiderme et/ou une diminution de l'épaisseur de la peau et/ou une perte d'élasticité et/ou de fermeté de la peau.

Les altérations de l'homéostasie épidermique se traduisent également par un aspect terne et/ou brouillé du teint de la peau.

Ce phénomène peut être accentué par la ménopause : les femmes se plaignent de ce que leur peau tire et devient sèche, voire de l'apparition d'une xérose. Les déficits hormonaux associés à la ménopause s'accompagnent notamment d'une baisse d'activité métabolique, ce qui pourrait aboutir à une diminution de la prolifération des kératinocytes et une augmentation de la différenciation épidermique.

Il est donc intéressant de disposer aussi de compositions capables de favoriser l'homéostasie de la peau afin de maintenir et/ou augmenter l'épaisseur de la peau et ainsi maintenir et/ou améliorer les propriétés mécaniques de la peau et/ou favoriser l'éclat du teint.

La présente invention ressort plus particulièrement de l'observation par les inventeurs qu'une association spécifique s'avère précisément efficace pour répondre à ces exigences et notamment via une action stimulatrice de l'expression de certains gènes. Avantageusement, elle permet de pallier au ralentissement de l'expression de certains gènes, notamment constaté au cours du vieillissement cutané comme pour le KRT6B.

En conséquence, la présente invention concerne, selon un premier de ses aspects, une composition cosmétique utile pour le soin et/ou le maquillage des matières kératiniques comprenant, dans un milieu physiologiquement acceptable, au moins un dérivé phytosphingosine-salicylate et au moins un lysat d'au moins un microorganisme du genre *Bifidobacterium species.*

Elle concerne également selon un autre de ses aspects, l'utilisation, notamment cosmétique, d'une association comprenant au moins un lysat d'au moins un microorganisme du genre *Bifidobacterium species* et au moins un dérivé phytosphingosine-salicylate pour stimuler l'expression d'au moins un gène choisi parmi les gènes KRT6B ou, KRT10 et involucrine.

Plus particulièrement, la présente invention concerne l'utilisation notamment cosmétique d'une association comprenant au moins un lysat d'au moins un microorganisme du genre *Bifidobacterium species* et au moins un dérivé phytosphingosine-salicylate pour stimuler l'expression d'au moins un gène choisi parmi les gènes KRTB6, KRT10 et involucrine, chez un individu sujet à un vieillissement cutané.

L'association considérée selon l'invention s'avère ainsi particulièrement avantageuse pour prévenir et/ou pallier la diminution d'expression observée avec l'âge pour le gène KRT6B..

On connaît certes de EP 0 043 128, la mise en oeuvre d'un lysat de microorganisme du genre *Bifidobacterium species* tel que le lysat Repair Complex CLR, mais uniquement à des fins de réparation de l'ADN des cellules de la peau.

Pour leur part, les documents suivants proposent la mise en oeuvre de microorganismes notamment probiotiques et pour l'essentiel à des fins de traitement de peaux sèches et/ou sensibles et troubles associés mais sous une forme distincte d'un lysat.

Ainsi, le document WO 02/28402 décrit que des microorganismes probiotiques peuvent avoir un effet bénéfique dans la régulation de réactions d'hypersensibilité cutanée comme les réactions inflammatoires et allergiques qui relèvent d'un processus immunologique. Les documents EP 1 609 463, EP 1 642 570, EP 1 731 137 et FR 2 876 029 décrivent des compositions associant un ou plusieurs microorganisme(s) probiotique(s) à un cation minéral pour le traitement des peaux sensibles. Quant au document PCT/FR2006/050768, il propose pour le traitement des peaux sensibles associées à une peau sèche, une association d'un microorganisme probiotique avec un acide gras polyinsaturé et/ou ester d'acide gras polyinsaturé. En conséquence, aucun de ces documents ne décrit la mise en oeuvre d'un lysat de microorganisme du genre *Bifidobacterium species,* et encore moins sous la forme associée considérée selon l'invention.

Pour ce qui est de la phytosphingosine et ses sels et, plus particulièrement, son chlorhydrate, ils sont déjà proposés dans le domaine de la dermatologie. En effet, la phytosphingosine est tout d'abord connue pour son activité antimicrobienne. Ainsi, la phytosphingosine est déjà mise à profit pour cette activité dans le traitement de l'acné et pour son activité d'inhibiteur de la croissance des microorganismes sur la peau (US 5,326,565 et EP 0 919 226). Plus récemment, des dérivés de sphingolipide et notamment des dérivés de type phytosphingosine-salicylate ont été décrits comme manifestant une aptitude à moduler la différenciation des kératinocytes. (G Paragh et al ; Exp Dermatol. 2008 Dec;17(12):1004-16. Pour sa part, le document US 5,882,665 propose de nouveaux dérivés phytosphingosine-salicylates décrits comme utiles à titre d'agents anti-acné, antibactériens, agents anti-rides, de même qu'agents éclaircissants de la peau.

Toutefois, à la connaissance des inventeurs l'effet bénéfique voire synergique manifesté par une association d'un dérivé phytosphingosine-salicylate et d'un lysat d'un microorganisme du genre *Bifidobacterium species* sur l'expression de certains gènes n'a jamais été constaté.

Comme le montre les exemples ci-après, une association conforme à l'invention, stimule manifestement l'expression du gène KRT6B, mais aussi des gènes KRT 10 et de l'involucrine. Cette action a notamment été vérifiée par qRT-PCR à partir d'un modèle de peau reconstruite Episkin®.

Au sens de l'invention, l'effet manifesté par l'association est qualifié de synergique dans la mesure où il s'avère supérieur à celui attendu de la simple superposition des effets respectifs du dérivé phytosphingosine-salicylate et du lysat d'un microorganisme du genre *Bifidobacterium species.*

Selon un autre de ses aspects, la présente invention concerne l'utilisation de l'association considérée ci-dessus pour renforcer la capacité de réparation et de régénération d'un épithélium, notamment d'un épiderme en particulier âgé.

Comme il ressort de ce qui précède, cet effet est notamment obtenu *via* la stimulation de l'expression de gène (s) ralentie naturellement lors du processus de vieillissement de la peau, et plus particulièrement *via* la stimulation de l'expression de la KRT6B.

Selon un de ses aspects, l'invention vise l'utilisation, notamment cosmétique d'une association comprenant au moins un lysat d'au moins un microorganisme du genre *Bifidobacterium species* et au moins un dérivé phytosphingosine-salicylate pour stimuler l'expression d'au moins un gène choisi parmi les gènes KRTB6, KRT10 et involucrine, en vue de renforcer la capacité de réparation et de régénération d'un épithélium, notamment d'un épiderme, en particulier âgé.

Selon encore un autre de ses aspects, la présente invention concerne un procédé de traitement notamment cosmétique, comprenant au moins l'administration à un individu sujet à un vieillissement cutané de l'association considérée ci-dessus.

Cette administration peut être effectuée, notamment par voie orale ou topique.

L'invention porte également sur l'utilisation cosmétique, notamment dans une composition contenant un milieu physiologiquement acceptable et dédiée à une application topique sur la peau d'une association selon l'invention pour augmenter l'épaisseur de la peau, favoriser l'éclat du teint, favoriser et/ou améliorer les propriétés mécaniques de la

peau, et/ou favoriser et/ou améliorer l'élasticité et/ou la fermeté de la peau.

**Microorganismes du genre *Bifidobacterium species***

Comme précisé précédemment, les microorganismes du genre *Bifidobacterium species* utilisés à titre d'actifs selon l'invention sont mis en oeuvre sous la forme d'un lysat.

Le lysat mis en oeuvre dans le cadre de la présente invention est tel que défini ci-après.

Un lysat désigne communément un matériau obtenu à l'issue de la destruction ou dissolution de cellules biologiques par un phénomène dit lyse cellulaire provoquant ainsi la libération des constituants biologiques intracellulaires naturellement contenus dans les cellules du microorganisme considéré.

Au sens de la présente invention, le terme lysat est utilisé indifféremment pour désigner l'intégralité du lysat obtenu par lyse du microorganisme concerné ou seulement une fraction de celui-ci.

Ainsi, l'invention concerne la mise en oeuvre d'un lysat de *Bifidobacterium species* et/ou une de ses fractions.

Le lysat mis en oeuvre est donc formé en tout ou partie des constituants biologiques intracellulaires et des constituants des parois et membranes cellulaires.

Plus précisément, il contient la fraction cytoplasmique cellulaire renfermant les enzymes tels que la déhydrogénase d'acide lactique, les phosphatases, les phosphokétolases, et transaldolases et les métabolites. A titre illustratif, les constituants des parois cellulaires sont notamment le peptidoglycane, la muréine ou mucopeptide et l'acide teichoique et les constituants des membranes cellulaires sont composés de glycérophospholipide.

Cette lyse cellulaire peut être accomplie par différentes technologies, telles que par exemple un choc osmotique, un choc thermique, par ultrasons, ou encore sous contrainte mécanique de type centrifugation.

Plus particulièrement, ce lysat peut être obtenu selon la technologie décrite dans le brevet US 4,464,362, et notamment selon le protocole suivant.

Un microorganisme de type *Bifidobacterium species* considéré est cultivé anaérobiquement dans un milieu de culture adéquat, par exemple selon les conditions décrites dans les documents US 4,464,362 et EP 0 043 128. Lorsque la phase stationnaire du développement est atteinte, le milieu de culture peut être inactivé par pasteurisation, par exemple à une température de 60 à 65 °C pendant 30 mn. Les microorganismes sont alors recueillis par une technique de séparation conventionnelle par exemple filtration membranaire, centrifugé et remis en suspension dans une solution stérile de NaCl à une concentration physiologique. Le lysat peut être obtenu par désintégration aux ultrasons d'un tel milieu afin d'en libérer les fractions cytoplasmiques, les fragments de paroi cellulaire et les produits issus du métabolisme. Puis tous les composants dans leur distribution naturelle sont ensuite stabilisés dans une solution aqueuse faiblement acide.

On obtient ainsi généralement un lysat possédant une concentration de l'ordre de 0,1 à 50 %, en particulier de 1 à 20 % et notamment environ 5 % en poids de matière(s) active(s) par rapport à son poids total.

Le lysat peut être mis en oeuvre sous différentes formes, sous la forme d'une solution ou sous une forme pulvérulente.

Convient plus particulièrement à l'invention, un microorganisme appartenant au genre *Bifidobacterium species* choisi parmi les espèces : *Bifidobacterium longum*, *Bifidobacterium bifidum*, *Bifidobacterium breve, Bifidobacterium animalis*, *Bifidobacterium lactis, Bifidobacterium infantis, Bifidobacterium adolescentis ou Bifidobacterium pseudocatenulatum*, et leurs mélanges.

Convient tout particulièrement à l'invention, l'espèce *Bifidobacterium longum.*

Il peut avantageusement s'agir du lysat enregistré sous le nom INCI : Bifidat ferment Lysate, sous le nom EINECS: Bifidobacterium longum, sous le N° EINECS: N° 306-168-4 et sous le N° CAS : N° 96507-89-0.

Le produit commercialisé sous la dénomination Repair Complex CLR® par la société K. RICHTER GmbH et qui est formé d'un lysat inactivé de l'espèce *Bifidobacterium longum*, entre dans le cadre de l'invention.Le lysat mis en oeuvre dans le cadre de la présente invention est tel que défini ci-dessus.

**Dérivé phytosphingosine-salicylate**

Naturellement, la phytosphingosine, présente dans le *stratum corneum*, correspond à l'une des trois bases sphingoïde naturellement présentes dans la peau.

Au sens de l'invention ces dérivés phytosphingosine-salicylate incluent les différentes formes ioniques des composés correspondants.

Un tel dérivé répond à la formule développée suivante :

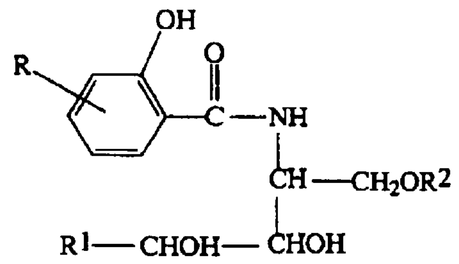

; (I)

dans laquelle

- R représente :
  - un atome d'hydrogène
  - un radical aliphatique linéaire ou ramifié, saturé ou insaturé, en $C_1$ à $C_{49}$, le cas échéant substitué par un radical hydroxyle, ou
  - un groupement $Y\text{-}O(C_aH_b)_m$-
    avec a étant un entier de 7 à 50, b un entier de 10 à 100, m est 0 ou 1 et Y représente H ou un acide gras en $C_{14}$-$C_{22}$ ayant la formule suivante : $-CO-(C_xH_yZ_z)CH_3$
    avec Z étant -OH ou un oxygène d'époxy, x un entier de 12 à 20, y un entier de 20 à 40, et z est 0 ou un entier de 1 à 4,

- $R^1$ représente un radical aliphatique linéaire ou ramifié, saturé ou insaturé, en $C_8$ à $C_{28}$, en particulier en $C_{10}$ à $C_{20}$, notamment en $C_{12}$ à $C_{18}$, le cas échéant substitué par un radical hydroxyle, et
- $R^2$ représente H, un radical phosphate, sulfate ou un sucre.

De tels dérivés sont plus particulièrement décrits dans le document EP 919 226.
Convient tout particulièrement à l'invention le dérivé de formule 1 dans laquelle R et $R^2$ figurent respectivement un atome d'hydrogène et $R^1$ un radical alkyle linéaire et saturé, notamment en $C_{14}$.
Ce dérivé répond à la formule suivante

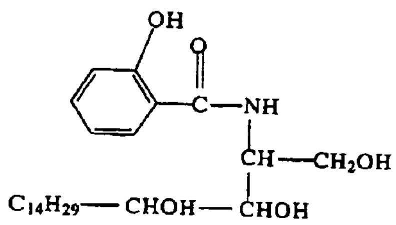

(V)

Un tel dérivé est notamment commercialisé par la société EVONICK GOLDSCHMIDT sous la dénomination Phytosphingosine SLC.
Le dérivé phytoshingosine-salicylate mis en oeuvre dans le cadre de la présente invention est tel que défini ci-dessus.
Les quantités respectives de dérivé phytosphingosine-salicylate et de lysat formant l'association selon l'invention, susceptibles, d'être mise en oeuvre, encore dites « quantités efficaces » sont, bien entendu, fonction de l'effet recherché et peuvent donc varier dans une large mesure.
Au sens de la présente invention, on entend désigner par l'expression "quantité efficace" la quantité minimale nécessaire à l'observation de l'effet attendu, à savoir un effet cosmétique ou un effet thérapeutique, étant entendu que les quantités efficaces nécessaires à l'obtention d'un effet cosmétique ou d'un effet thérapeutique peuvent être, le cas échéant, identiques ou différentes.
Pour donner un ordre de grandeur, chacun des deux composés formant l'invention peuvent être présent en une quantité représentant de 0,0001 % à 50 % du poids total de la composition, en particulier en une quantité représentant de 0,001 % à 10 % du poids total de la composition.
Plus particulièrement, dans les compositions selon l'invention, l'actif formant le lysat et appartenant au genre

*Bifidobacterium species* peut être mis en oeuvre à raison d'au moins 0,001 % (exprimé en poids sec), en particulier à raison de 0,01 à 20 % et plus particulièrement à raison de 0,01 à 15 % en poids sec de matière active par rapport au poids total du support ou de la composition le contenant.

Pour sa part, dans les compositions selon l'invention, le dérivé phytosphingosine-salicylate peut être formulé dans une composition à raison d'au moins 0,0001 % (exprimé en poids sec), en particulier à raison de 0,001 à 20 % et plus particulièrement à raison de 0,05 à 2 % en poids sec de matière active par rapport au poids total du support ou de la composition le contenant.

**Composition selon l'invention**

Les compositions considérées selon l'invention peuvent être cosmétiques ou pharmaceutiques, en particulier dermatologiques.

Au sens de l'invention, on entend par « prévention d'un trouble », le ralentissement de survenue des troubles associés au vieillissement cutané, notamment tel que défini précédemment.

Il est entendu que l'ensemble des compositions considérées selon l'invention, mettent en oeuvre un milieu physiologiquement acceptable.

Au sens de la présente invention, on entend désigner par "milieu physiologiquement acceptable", un milieu convenant à l'application d'une composition sur une matière kératinique, en particulier la peau.

Selon un mode de réalisation particulier, une composition conforme à l'invention peut comprendre, en outre, au moins un agent complémentaire actif au niveau cutané.

Les quantités des différents constituants susceptibles d'être annexés à l'association selon l'invention sont celles classiquement utilisées dans les domaines considérés.

*- Actifs annexes*

Comme exemples d'actifs annexes utilisables dans le cadre de la présente invention, il peut être fait mention des actifs permettant d'améliorer l'état de la peau, tels que des actifs hydratants ou humidifiants ou des agents actifs permettant d'améliorer la barrière lipidique naturelle, tels que des céramides, des sulfates de cholestérol et/ou des acides gras et leurs mélanges.

Il peut également être possible d'utiliser des enzymes ayant une activité sur la peau, telles que des protéases, des lipases, des cérébrosidases, des amidases et/ou des mélanases et leurs mélanges.

Il est également possible d'utiliser des actifs de type microorganismes notamment probiotiques, comme ceux décrits dans les demandes WO 2006/000992 et WO 2006/037922.

Au sens de la présente invention, on entend par "microorganisme probiotique", un microorganisme vivant qui, lorsqu'il est consommé en quantité adéquate, a un effet positif sur la santé de son hôte selon le « Joint FAO/WHO Expert Consultation on Evaluation of Health and Nutritional Properties of Probiotic in Food Including Powder Milk with Live Lactic Acid Bacteria, 6 octobre 2001 », et qui peut en particulier améliorer l'équilibre microbien intestinal.

Les microorganismes convenant à l'invention peuvent être choisis notamment parmi les ascomycètes telles que Saccharomyces, Yarrowia, Kluyveromyces, Torulaspora, Schizosaccharomyces pombe, Debaromyces, Candida, Pichia, Aspergillus et Penicillium, des bactéries du genre Bifidobacterium, Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus et Lactobacillus et leurs mélanges.

Comme ascomycètes convenant tout particulièrement à la présente invention, on peut en particulier citer *Yarrowia lipolitica et Kluyveromyces lactis*, de même que *Saccharomyces cereviseae*, *Torulaspora*, *Schizosaccharamyces pombe*, *Candida* et *Pichia*.

Des exemples spécifiques de microorganismes probiotiques sont *Bifidobacterium bifidum*, *Bifidobacterium infantis*, *Lactobacillus acidophilus*, *Lactobacillus alimentarius*, *Lactobacillus curvatus*, *Lactobacillus delbruckii subsp. Lactis*, *Lactobacillus gasseri*, *Lactobacillus johnsonii*, *Lactobacillus reuteri*, *Lactobacillus rhamnosus* (*Lactobacillus GG*), *Lactobacillus sake*, *Lactococcus lactis*, *Streptococcus thermophilus*, *Staphylococccus carnosus, et Staphylococcus xylosus* et leurs mélanges.

Plus particulièrement, il s'agit de microorganismes probiotiques issus du groupe des bactéries lactiques, comme notamment les *Lactobacillus* et/ou les *Bifidobacterium*. A titre illustratif de ces bactéries lactiques, on peut plus particulièrement citer les *Lactobacillus johnsonii*, *Lactobacillus reuteri*, *Lactobacillus rhamnosus*, *Bifidobacterium bifidum*, *Bifidobacterium breve*, *Bifidobacterium animalis*, *Bifidobacterium lactis*, *Bifidobacterium longum*, *Bifidobacterium infantis*, *Bifidobacterium adolescentis ou Bifidobacterium pseudocatenulatum* et leurs mélanges.

Une souche de *Bifidobacterium lactis* peut être obtenue chez Hansen (Chr. Hansen A/S, 10-12 Boege Alle, P.O. Box 407, DK-2970 Hoersholm, Danemark) sous l'appellation Bb 12.

Le ou les microorganisme(s) peu(ven)t être inclus dans la composition selon l'invention sous une forme vivante,

semi-active ou inactivée, morte.

Il(s) peu(ven)t également être inclus sous forme de fractions de composants cellulaires ou sous la forme de métabolites. Le ou le(s) microorganisme(s), métabolite(s) ou fraction(s) peu(ven)t également être introduit(s) sous la forme d'une poudre lyophilisée, d'un surnageant de culture et/ou le cas échéant sous une forme concentrée.

Dans le cas particulier des compositions topiques, il peut être avantageux de mettre en oeuvre ces microorganismes sous forme inactivée, voire morte.

En ce qui concerne les microorganismes probiotiques, ce sont les genres bactériens et de levures suivants qui sont généralement utilisés :

- Les bactéries lactiques : qui produisent par fermentation du sucre de l'acide lactique. Suivant leur morphologies ont les divisent en deux groupes :
  - *Lactobacillus species : acidophilus* (LC1, NCFB 1748) ; *amylovorus*, *casei (Shirota), rhamnosus* (soude GG), *brevis, crispatus, delbrueckii (subsp bulgaricus, lactis), fermemtum, helveticus, gallinarum, gasseri, lohnsonii, paracasei, plantarum, reuteri, rhamnosus, salivarius),*
  - *Gocci : Enterocossus (faecalis, faeciul), Lactococcus lactis (subspp lactis ou cremoris), Leuconstoc mesenteroides subsp dextranicum, Pediococcus acidilactici* (alimentation animale), *Sporolactobacillus inulinus, Streptococcus salvarius subsp. Thermophilus*
- Les *bifidobactéries* ou *Bifidobacterium species : Bifidobacterium adodescentis ; animalis, bifidum, breve, lactis, longum, infantis,*
- Les *levures : Saccharomyces (cerevisiae ou encore boulardii),*
- Les autres bactéries sporulées : *Bacillus (cereus var toyo ou subtilis), Bacillus coagulans, B licheniformis, Escherichia coli strain nissle, Propionibacterium freudenreichii.*

Les bactéries lactiques et les bifidobactéries sont les probiotiques le plus souvent utilisés.

Des exemples spécifiques de microorganismes probiotiques convenant tout particulièrement à l'invention sont *Bifidobacterium bifidum, Bifidobacterium infantis, Bifidobacterium longum, Lactobacillus acidophilus, Lactobacilllus alimentarius, Lactobacillus casei subsp. Casei, Lactobacillus casei Shirota, Lactobacillus paracasei, Lactobacillus curvatus, Lactobacillus delbruckii subsp. Lactis, Lactobacillus gasseri, Lactobacillus johnsonii, Lactobacilllus reuteri, Lactobacillus rhamnosus (Lactobacillus GG), Lactobacillus sake, Lactococcus lactis, Streptococcus thermophilus, Staphylococcccus carnosus,* et *Staphylococcus xylosus* et leurs mélanges.

Plus particulièrement, il s'agit de microorganismes probiotiques issus du groupe des bactéries lactiques, comme notamment les *Lactobacillus* et/ou les *Bifidobacterium.* A titre illustratif de ces bactéries lactiques, on peut plus particulièrement citer les *Lactobacillus johnsonii, Lactobacillus reuteri, Lactobacillus rhamnosus, Lactobacillus paracasei, Lactobacillus casei* ou *Bifidobacterium bifidum, Bifidobacterium breve, Bifidobacterium longum, Bifidobacterium animalis, Bifidobacterium lactis, Bifidobacterium infantis, Bifidobacterium adolescentis* ou *Bifidobacterium pseudocatenulatum* et leurs mélanges.

Les espèces convenant tout particulièrement sont les *Lactobacillus johnsonii, Lactobacillus paracasei, Bifidobacterium adolescentis, Bifidobacterium longum* et *Bifidobacterum Lactis* NCC 2818 (encore désigné Bb12 ATCC 27536) respectivement déposées suivant le traité de Budapest avec l'Institut Pasteur (28 rue du Docteur Roux, F-75024 Paris cedex 15) les 30/06/92, 12/01/99, 15/04/99, 15/04/99, 07/06/05 sous les désignations suivantes CNCM I-1225, CNCM I-2116, CNCM I-2168 et CNCM I-2170 et CNCM I-3446, et le genre Bifidobacterium longum (BB536). La souche de Bifidobacterium lactis CNCM I-3446 peut être obtenue chez Hansen (Chr. Hansen A/S, 10-12 Boege Alle, P.O. Box 407, DK-2970 Hoersholm, Danemark).

Comme autres exemples d'agents actifs convenant à la mise en oeuvre de la présente invention figurent : des actifs analgésiques, des actifs anti-levures, des actifs anti-bactériens, des actifs antiparasitaires, des actifs antifongiques, des actifs antiviraux, des actifs anti-inflammatoires stéroïdiens, des actifs anesthésiques, des actifs antiprurigineux, des actifs kératolytique, des actifs anti-radicaux libres, des actifs antiséborrhéiques, des actifs antipelliculaires, des actifs antiacnéiques, des actifs visant à prévenir le vieillissement de la peau et/ou à améliorer son état, des actifs anti-âge, des actifs anti-dermatites, des actifs anti-irritants, des actifs immunomodulateurs, des actifs pour le traitement de la peau sèche, des actifs anti-transpirants, des actifs anti-psoriatiques, des actifs anti-histaminiques, des actifs cicatrisants, des actifs auto-bronzants, des antioxydants tels que le thé vert ou des fractions actives de celui-ci, la glycérine, la laponite, la caféine, des huiles essentielles aromatiques, d des actifs dépigmentants, des actifs exfoliants, des liporégulateurs, des actifs adoucissants, rafraîchissants, déodorants, insensibilisants, blanchissants, nourrissants, des actifs diminuant la différenciation et/ou la prolifération et/ou la pigmentation cutanée et leurs mélanges.

Les actifs annexes pourront aussi être choisis parmi, les agents améliorant la fonction barrière, les agents dermodécontractants, les agents anti-glycation, les agents stimulant la synthèse de macromolécules dermiques et/ou

épidermiques et/ou empêchant leur dégradation, les agents stimulant la prolifération des fibroblastes ou des kératinocytes et/ou la différenciation des kératinocytes, les agents favorisant la maturation de l'enveloppe cornée, les inhibiteurs de NO-synthases, les antagonistes des récepteurs périphériques des benzodiazépines (PBR), les agents augmentant l'activité de la glande sébacée, les agents stimulant le métabolisme énergétique des cellules, les agents tenseurs, les agents liporestructurants, les agents amincissants, les agents favorisant la microcirculation cutanée, les agents apaisants, les sébo-régulateurs ou anti-séborrhéiques, les agents astringents, les agents anti-inflammatoires, les agents anti-acné.

Parmi les actifs et notamment les actifs anti-âge pouvant être utilisés dans le cadre de la présente invention, peuvent être cités à titre d'exemples non limitatifs : les extraits de protéines de soja comme celui commercialisé par la société SILAB sous la dénomination commerciale Raffermine®), l'adenosine ou adénosine de synthèse comme celle commercialisée par Pharma Waldoff, le pro-xylane des extraits de graines de seigle comme ceux commercialisés par Silab sous la dénomination Coheliss, des extraits d'alfalfa (luzerne) comme ceux commercialisés par Silab sous la dénomination Vitanol, le tétrapeptide (N-acétyl-Gln-ASP-VAL-HIS) comme celui commercialisé par Cognis sous la référence Dermican LS 9745, des di et tri-peptides de riz comme ceux disponibles auprès de la société Silab sous la référence Nutriskin, des extraits de graines de vigna aconitifolia comme ceux commercialisés par la société Cognis sous les références Vitoptine LS 9529 et Vit-A-Like LS9737, des extraits d'*Euglena gracilis* comme ceux disponibles auprès de Sederma sous la référence Chronodyn, des acides aminés de blé couplés à l'acide palmitique commercialisé sous la référence Deepline PVB (Lipacide PVB) par Seepic, des extraits de myrtille comme Herbasol Myrtille Extract de la société Cosmetochem, des extraits de racine de gingembre, des extraits aqueux de Shii Take (LEntinus edodes) comme Fermiskin de Silab, l'antarcticine comemrcialisé par Lipotec, des extraits de *Punica granatum*, l'argireline SC36 (hexapeptide (Acétyl-Glu-Glu-Meth-Glu-Arg-Arg-Amide) de la société Lipotec, un extarit d'avoine comme celui commercialisé par Silab sous la référence Reductine, un extrait de riz pourpre comme le Purple rice extract de la société Oryza, des dispersions de cellules de fleurs de vigne comme le Fiber Booster Sequoia vitis flower de la société Naolys, de l'acide férullique comme Oryzaferulix de la société Oryza Oil and fat, des extraits de Voandzeia subterranea (Bambara) comme Filadyn LS 9397 de la société Cognis, des extraits de graines de soja comme le produit Elhibin commercialisé par la société Pentapharm, un extrait du fruit Prunus domestica hydrolysé comme la référence Clairju de la société Ichimaru Pharco.

En particulier, parmi les autres agents actifs au niveau cutané convenant à l'invention on peut mentionner des agents actifs hydratants et/ou desquamants tel que le glycol, l'urée ou ses dérivés, l'HEPES, des chélateurs, des détergents, des dérivés de l'acide jasmonique, et leur mélange.

L'homme du métier choisira le ou lesdits actifs en fonction de l'effet recherché sur les matières kératiniques.

- <u>Formes galéniques</u>

Pour une administration par la voie orale, une composition de l'invention peut se présenter sous toutes les formes adaptées, particulièrement sous forme d'une solution buvable, d'un sirop, d'un comprimé, d'une dragée, d'une gélule, d'une capsule ou encore d'un aliment nutritionnel ou d'un complément nutritionnel.

Une composition selon l'invention peut comprendre en outre au moins un excipient approprié adapté à l'administration par voie orale.

L'association considérée selon l'invention de même que toute composition la contenant peut avantageusement être administrée par voie topique.

Une composition dédiée à une administration topique peut notamment se présenter sous la forme d'une solution aqueuse, hydroalcoolique ou huileuse, d'une dispersion du type solution ou dispersion du type lotion ou sérum, d'une émulsion de consistance liquide ou semi-liquide du type lait, obtenue par dispersion d'une phase grasse dans une phase aqueuse (H/E) ou inversement (E/H), ou d'une suspension ou émulsion de consistance molle, semi-solide ou solide du type crème, de gel aqueux ou anhydre, ou encore d'une microémulsion, de microcapsules, de microparticules, ou de dispersions vésiculaires de type ionique et/ou non ionique.

Le pH d'une composition selon l'invention, lorsqu'elle comprend au moins une phase aqueuse (ex : solutions aqueuses, émulsions...), est de préférence compris entre 4 et 9, de préférence entre 4 et 7, avantageusement de 5 à 6, et en particulier un pH de 5,5.

La composition selon l'invention peut être plus ou moins fluide et avoir l'aspect d'une crème blanche ou colorée, d'une pommade, d'un lait, d'une lotion, d'un sérum, d'une pâte, ou d'une mousse. Elle peut éventuellement être appliquée sur la peau sous forme d'aérosol. Elle peut également se présenter sous forme solide, et par exemple sous forme de stick. Elle peut être utilisée comme produit de soin, mais aussi de toilette et/ou de maquillage.

Cette composition peut constituer un masque, une crème de nettoyage, de protection, de traitement ou de soin pour le visage, pour les mains, pour les pieds, ou pour le corps (par exemple crèmes de jour, crèmes de nuit, crèmes démaquillantes, crèmes de fond de teint, crèmes anti-solaires), un lait de démaquillage, une lotion, gel ou mousse pour le soin de la peau, comme une lotion de nettoyage.

D'une manière générale, toute composition de l'invention peut être appliquée sur la peau (sur toute zone cutanée du corps) ou sur les muqueuses (buccale, jugale, gingivale, génitale, conjonctivale, ...).

De façon connue, une composition cosmétique peut contenir également des adjuvants habituels dans le domaine cosmétique, tels que des gélifiants hydrophiles ou lipophiles, des additifs hydrophiles ou lipophiles, des conservateurs, des antioxydants, des solvants, des parfums, des charges, des filtres UV (solaires), des absorbeurs d'odeurs et des matières colorantes.La teneur et la nature des ingrédients mis en oeuvre dans les compositions de l'invention sont ajustées par l'homme de l'art de sorte à ne pas affecter substantiellement l'effet de l'association considérée selon l'invention.

Une association selon l'invention peut s'avérer particulièrement avantageuse pour améliorer l'hydratation, l'homéostasie cutanée et en particulier épidermique, la fonction barrière cutanée, prévenir et/ou traiter les signes de vieillissement épidermiques tels que par exemple les rides, ridules, perte de fermeté, d'élasticité, densité et/ou de tonicité d'un épiderme.

Les troubles cutanés plus particulièrement visés par la présente invention peuvent être donc la peau sèche, la peau très sèche, et chez les individus dont la peau présente un aspect âgé, notamment chez les femmes au-delà de 45 ans et/ou ménopausées, voire très âgées.

Comme précisé précédemment, la présente invention concerne également un procédé, notamment cosmétique comprenant au moins l'administration à un individu, sujet à un vieillissement cutané, d'au moins une association selon l'invention.

Un tel procédé de traitement peut être mis en oeuvre notamment par administration topique ou orale, journalière par exemple, de l'association considérée selon l'invention.

Un procédé selon l'invention peut comprendre une administration unique. Selon un autre mode de réalisation, l'administration est répétée par exemple 2 à 3 fois quotidiennement sur une journée ou plus et généralement sur une durée prolongée d'au moins 4 semaines, voire 4 à 15 semaines, avec le cas échéant une ou plusieurs périodes d'interruption.

Dans la description et dans les exemples suivants, sauf indication contraire, les pourcentages sont des pourcentages en poids et les plages de valeurs libellées sous la forme « entre ... et ... » incluent les bornes inférieure et supérieure précisées.

L'exemple et la figure ci-après sont présentés à titre illustratif et non limitatif du domaine de l'invention.

**<u>Exemple 1</u>**

Dans cet exemple, le lysat utilisé est le produit commercialisé sous la dénomination Repair Complex CLR® par la société K. RICHTER GmbH et qui est formé d'un lysat inactivé de l'espèce *Bifidobacterium longum* et le dérivé phytosphingosine-salicylate est celui commercialisé par la société EVONICK GOLDSCHMIDT sous la dénomination Phytosphingosine SLC.

Les effets des quatre compositions suivantes ont été testés sur la différenciation d'épidermes reconstruits Episkin®.

- A : support formulatoire, (Alcool éthylique (3 %) et eau (97 %)),
- B : lysat, (Alcool éthylique (3 %), eau (87 %), lysat (10 %)),
- C : phytosphingosine-salicylate, (Alcool éthylique (3 %), eau (96,998 %), Phytosphingosine-salicylate (0,002 %)), et
- D : association conforme à l'invention = (0,002 % phytosphingosine-salicylate + 10 % lysat + Alcool éthylique (3 %) + eau (86,998 %)).

Des épidermes reconstruits Episkin® J6 ont été placés en plaques 12 puits contenant 2 ml d'un milieu de maintenance et cultivés à 37 °C et 5 % $CO_2$ pendant 24 heures. Après incubation, les épidermes ont été traités en topique avec les compositions à tester (50µl/épiderme reconstruit) et incubés pendant 6 heures.

Ces effets ont été caractérisés par l'analyse de l'expression des gènes cytokeratin 6B (K6B), cytokeratin 10 (K10) et involucrine (INV) en utilisant une méthode de PCR en temps réel (PCR quantitative, qPCR) 6 heures après traitement.

**<u>1- Protocoles retenus</u>**

**a) Analyse de l'expression différentielle**

L'expression des marqueurs sélectionnés a été évaluée par RT-qPCR sur les ARN messagers extraits des épidermes reconstruits Episkin® de chaque traitement (les duplicates ont été poolés avant l'extraction de l'ARN) 6, 24 et 48 heures après traitement.

**b) Reverse Transcription**

- Extraction des ARN totaux de chaque échantillon à l'aide de Tri-Reagent.
- Elimination des traces d'ADN potentiellement contaminant par traitement avec le système DNA-free (Ambion réf. 1906). Quantification des ARN à l'aide du Nanovue (Amersham).
- Réalisation de la réaction de reverse-transcription de l'ARNm en présence de l'amorce oligo(dT) et de l'enzyme Superscript II (Gibco).
- Quantification de l'ADNc obtenu à l'aide de Nanovue (Amersham) et ajustement des ADNc à 10 ng/gl.

**c) PCR Quantitative**

Les réactions de PCR (polymerase chain reactions) ont été réalisées par PCR quantitative avec le système « Lie Cycler » (Roche Molecular Systems Inc.) Ce système d'analyse permet de réaliser des réactions de PCR rapides et performantes, moyennant une mise au point préalable des conditions d'analyse des différents primers. Il est formé de deux composants principaux :

- *un thermocycleur optimisé :* permettant des transferts thermiques extrêmement rapides.
- *un fluorimètre :* permettant de mesurer en continu l'intensité de fluorescence incorporée dans l'ADN (détection à 521 nm).

Des couples de sondes spécifiques des gènes étudiés ont été utilisés permettant l'amplification des fragments spécifiques suivants :

| **Nom du gène (protéine codée)** | **Abréviation** | **Gene Bank** | **cDNA pb** |
|---|---|---|---|
| Liver glyceraldehyde 3-phosphate | G3PDH | **NM** 002046 | 269 |
| Cytokeratin 6B | K6B | **NM** 005555 | 277 |
| Cytokeratin 10 | K10 | **NM** 000421 | 236 |
| Involucrine | **INV** | **NM** 005547 | **251** |

Le mélange réactionnel (10 μl final) pour chaque échantillon est le suivant :

- 2,5 μg d'ADNc
- Amorces des différents marqueurs utilisés
- Mélange réactionnel (Roche) contenant l'enzyme taq DNA polymérase, le marqueur SR Green 1 (fluorophore qui s'intercale dans l'ADN double brin, au cours de l'étape d'élongation) et du $MgCl_2$.

**d) Traitement des données de PCR quantitative**

L'incorporation de fluorescence dans l'ADN amplifié est mesurée en continu au cours des cycles de PCR. Ce système permet d'obtenir des courbes de mesure de la fluorescence en fonction des cycles de PCR et d'évaluer ainsi une valeur d'expression relative pour chaque marqueur.

Le nombre de cycles est déterminé à partir des points de « sortie » des courbes de fluorescence. Pour un même marqueur analysé, plus un échantillon sort tard (nombre de cycles élevé), plus le nombre initial de copies de l'ARNm est faible.

La valeur de l'expression relative est exprimée en unités arbitraires selon la formule suivante :

$$(1/2^{\text{nombre de cycles}}) \times 10^6$$

**e) Standardisation des effets observés sur l'expression des gènes**

Pour une interprétation standardisée, le tableau de classification suivant a été retenu :

| % d'expression relative par rapport au témoin | Classification de l'effet observé |
|---|---|
| > 150% et < 200 % | Stimulation modérée, à confirmer |
| > 200 % | Stimulation nette |
| > 300 % | Forte stimulation |
| < 65% et > 50 % | Inhibition modérée |
| < 50% et > 30 % | Inhibition nette |
| < 30 % | Forte Inhibition |

**2- RESULTATS**

Les cinétiques d'expression des trois études relatives aux marqueurs sont présentées dans le Tableau 1 ci-après et illustrées en figure 1.

Les résultats sont rapportés à la quantité de messager G3PDH (gène de référence) et exprimés en % du témoin non traité.

| Traitement | | K6B | K10 | INV |
|---|---|---|---|---|
| 6 heures | **Témoin** | 100 | 100 | 100 |
| | **Composition A** | 117 | 121 | 176 |
| | **Composition** B **(lysat)** | 176 | 102 | 153 |
| | **Composition C (phytosphingosine)** | 158 | 122 | 146 |
| | **Composition D (association)** | 207 | 147 | 265 |

Dans les *conditions* expérimentales de cet essai, il apparaît que seule l'association conforme à l'invention augmente au terme de 6 heures l'expression des gènes codants pour la cytokeratine 6B, cytokeratine 10 et l'involucrine.

**Exemple 2**

Les composés sont, selon le cas, cités en noms chimiques ou en noms CTFA (International Cosmetic Ingredient Dictionary and Handbook).

Composition pour le soin du visage sous forme de gel aqueux

| | % en poids |
|---|---|
| Phytosphingosine-SLC® | 0,002 % |
| Repair Complex CLR® | 10 % |
| Hyaluronate de sodium | 0,4 % |
| Gomme de xanthane | 0,1 % |
| Octane-1,2 diol | 0,3 % |
| Acide Ethylène Diamine Trétraacétique, Sel disodique | 0,099 % |
| Glycérine | 5% |
| Acide citrique | 0,015% |
| Alcool éthylique | 4,6% |
| Acide 4-(2-hydroxyéthyl)-pipérazin-1-éthanesulfonique | 1 % |
| Corps gras | 0,016% |
| Emulsionnant | 0,099 % |
| Charge | 0,2 % |

(suite)

| | % en poids |
|---|---|
| Conservateurs | 0,55 % |
| Solvant | 1 % |
| Parfum | 0,005 % |
| Eau | QSP 100% |

## Revendications

**1.** Composition cosmétique utile pour le soin et/ou le maquillage des matières kératiniques comprenant, dans un milieu physiologiquement acceptable, au moins un dérivé phytosphingosine-salicylate et au moins un lysat d'au moins un microorganisme du genre *Bifidobacterium species,* ledit dérivé phytosphingosine-salicylate répondant à la formule suivante :

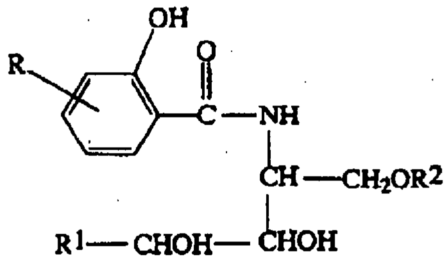

; (I)

dans laquelle

- R représente :

  - un atome d'hydrogène
  - un radical aliphatique linéaire ou ramifié, saturé ou insaturé, en $C_1$ à $C_{49}$, le cas échéant substitué par un radical hydroxyle, ou
  - un groupement $Y\text{-}O(C_aH_b)_m\text{-}$
  avec a étant un entier de 7 à 50, b un entier de 10 à 100, m est 0 ou 1 et Y représente H ou un acide gras en $C_{14}$-$C_{22}$ ayant la formule : $-CO-(C_xH_yZ_z)CH_3$
  avec Z étant -OH ou un oxygène d'époxy, x un entier de 12 à 20, y un entier de 20 à 40, et z est 0 ou un entier de 1 à 4,

- $R^1$ représente un radical aliphatique linéaire ou ramifié, saturé ou insaturé, en C8 à C28, le cas échéant substitué par un radical hydroxyle, et
- $R^2$ représente H, un radical phosphate, sulfate ou un sucre.

**2.** Composition selon la revendication précédente dans laquelle ledit microorganisme du genre *Bifidobacterium species* est choisi parmi *Bifidobacterium longue, Bifidobacterium bifidum, Bifidobacterium breve, Bifidobacterium animalis, Bifidobacterium lactis, Bifidobacterium infantis, Bifidobacterium adolescentis, Bifidobacterium pseudocatenulatum* et leurs mélanges.

**3.** Composition selon l'une quelconque des revendications précédentes dans laquelle le microorganisme du genre *Bifidobacterium species* est le *Bifidobacterium longum.*

**4.** Composition selon l'une quelconque des revendications précédentes dans laquelle ledit lysat est mis en oeuvre à raison d'au moins 0,001 % (exprimé en poids sec), en particulier à raison de 0,01 à 20 % et plus particulièrement à raison de 0,1 à 15 % en poids sec de matière active par rapport au poids total de la composition.

**5.** Composition selon la revendication 1, dans laquelle R et $R^2$ figurent respectivement un atome d'hydrogène et $R^1$

un radical alkyle linéaire et saturé.

6. Composition selon l'une quelconque des revendications précédentes dans laquelle le dérivé phytosphingosine-salicylate est

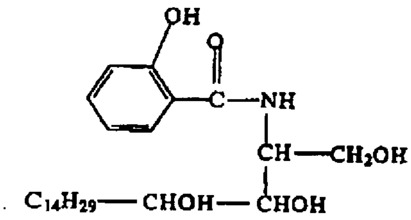

(V)

ou l'un de ses sels.

7. Composition selon l'une quelconque des revendications précédentes dans laquelle le dérivé phytosphingosine-salicylate est mis en oeuvre à raison d'au moins 0,0001 % (exprimé en poids sec), en particulier à raison de 0,001 à 20 °% et plus particulièrement à raison de 0,05 à 2 % en poids sec de matière active par rapport au poids total de la composition.

8. Composition selon l'une quelconque des revendications précédentes dans laquelle ladite composition est dédiée à une administration par voie topique.

9. Utilisation cosmétique d'une association comprenant au moins un lysat d'au moins un microorganisme du genre *Bifidobacterium species* et au moins un dérivé phytosphingosine-salicylate tel que défini en revendications 1, 5 ou 6 pour stimuler l'expression d'au moins un gène choisi parmi les gènes KRTB6, KRT10 et involucrine, chez un individu sujet à un vieillissement cutané.

10. Utilisation cosmétique d'une association comprenant au moins un lysat d'au moins un microorganisme du genre *Bifidobacterium species* et au moins un dérivé phytosphingosine-salicylate tel que défini en revendications 1, 5 ou 6 pour renforcer la capacité de réparation et de régénération d'un épithélium, notamment d'un épiderme en particulier âgé.

11. Utilisation selon l'une quelconque des revendications 9 et 10 dans laquelle ledit lysat est tel que défini en revendications 2 à 4.

12. Procédé de traitement cosmétique, comprenant au moins l'administration à un individu sujet à un vieillissement cutané, d'au moins une association comprenant au moins un lysat d'au moins un microorganisme du genre *Bifidobacterium species* et au moins un dérivé phytosphingosine-salicylate tel que défini en revendications 1, 5 ou 6.

## Claims

1. Cosmetic composition that is of use for the care of and/or making up keratin materials, comprising, in a physiologically acceptable medium, at least one phytosphingosine salicylate derivative and at least one lysate of at least one microorganism of the genus *Bifidobacterium species,* said phytosphingosine salicylate derivative corresponding to the following formula:

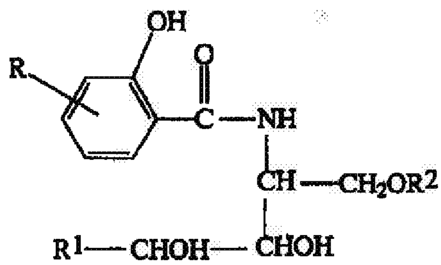

(I)

in which:

R represents:

- a hydrogen atom,
- a saturated or unsaturated, linear or branched $C_1$ to $C_{49}$ aliphatic radical, where appropriate substituted with a hydroxyl radical, or
- a group $Y\text{-}O(C_aH_b)_m\text{-}$,
with a being an integer from 7 to 50, b an integer from 10 to 100, m is 0 or 1 and Y represents H or a $C_{14}$-$C_{22}$ fatty acid having the formula: $-CO-(C_xH_yZ_z)\ CH_3$,
with Z being -OH or an epoxy oxygen, x an integer from 12 to 20, y an integer from 20 to 40, and z is 0 or an integer from 1 to 4;

- $R^1$ represents a saturated or unsaturated, linear or branched $C_8$ to $C_{28}$ aliphatic radical, where appropriate substituted with a hydroxyl radical, and
- $R^2$ represents H, a phosphate radical, a sulphate radical or a sugar

2. Composition according to the preceding claim, in which said microorganism of the genus *Bifidobacterium species* is chosen from *Bifidobacterium longum*, *Bifidobacterium bifidum*, *Bifidobacterium breve, Bifidobacterium animalis, Bifidobacterium lactis, Bifidobacterium infantis, Bifidobacterium adolescentis* and *Bifidobacterium pseudocatenulatum*, and their mixtures thereof.

3. Composition according to either one of the preceding claims, in which the microorganism of the genus *Bifidobacterium species* is *Bifidobacterium longum.*

4. Composition according to any one of the preceding claims, in which said lysate is used in a proportion of at least 0.001% (expressed as dry weight), in particular in a proportion of from 0.01% to 20%, and more particularly in a proportion of from 0.1% to 15% by dry weight of active material, relative to the total weight of the composition.

5. Composition according to Claim 1, in which R and $R^2$ are, respectively, a hydrogen atom and $R^1$ a saturated, linear alkyl radical.

6. Composition according to any one of the preceding claims, in which the phytosphingosine salicylate derivative is:

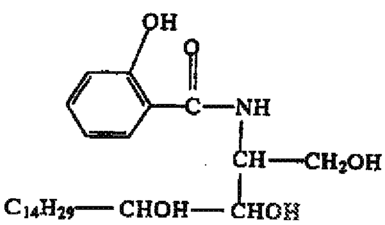

(V)

or a salt thereof.

7. Composition according to any one of the preceding claims, in which the phytosphingosine salicylate derivative is used in a proportion of at least 0.0001% (expressed as dry weight), in particular in a proportion of from 0.001% to

20%, and more particularly in a proportion of from 0.05% to 2% by dry weight of active material, relative to the total weight of the composition.

**8.** Composition according to any one of the preceding claims, in which said composition is devoted to topical administration.

**9.** Cosmetic use of a combination comprising at least one lysate of at least one microorganism of the genus *Bifidobacterium species* and at least one phytosphingosine salicylate derivative as defined in Claims 1, 5 or 6, for stimulating the expression of at least one gene chosen from the KRTB6, KRT10 and involucrin genes in an individual subject to skin aging.

**10.** Cosmetic use of a combination comprising at least one lysate of at least one microorganism of the genus *Bifidobacterium species* and at least one phytosphingosine salicylate derivative as defined in Claims 1, 5 or 6, for reinforcing the repair and regeneration capacity of an epithelium, especially an epidermis, in particular an aged epidermis.

**11.** Use according to either one of Claims 9 and 10, in which said lysate is as defined in Claims 2 to 4.

**12.** Cosmetic treatment method, comprising at least the administration, to an individual subject to skin aging, of at least one combination comprising at least one lysate of at least one microorganism of the genus *Bifidobacterium species* and at least one phytosphingosine salicylate derivative as defined in Claims 1, 5 or 6.

## Patentansprüche

**1.** Kosmetische Zusammensetzung, die für die Pflege und/oder das Schminken nützlich ist, aus Keratinmaterialien, die in einer physiologisch verträglichen Umgebung mindestens ein Phytosphingosin-Salicylat-Derivat und mindestens ein Lysat mindestens eines Mikroorganismus der Gattung *Bifidobacterium species* umfasst, wobei das Phytosphingosin-Salicylat-Derivat der folgenden Formel entspricht:

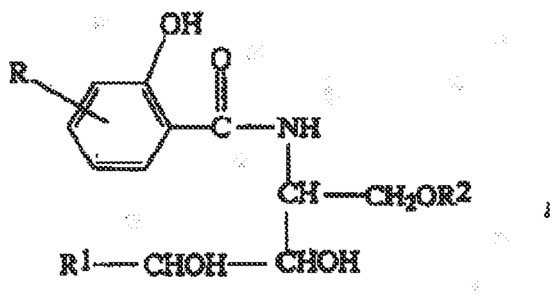
(I)

in der

- R darstellt:

  - ein Wasserstoffatom
  - einen aliphatischen linearen oder verzweigten, gesättigten oder ungesättigten $C_1$-$C_{49}$-Rest, der gegebenenfalls mit einem Hydroxylrest substituiert ist, oder
  - eine Gruppe Y-O($C_aH_b$)$_m$-
  wobei a eine ganze Zahl von 7 bis 50 ist, b eine ganze Zahl von 10 bis 100 ist, m 0 oder 1 ist und Y der eine $C_{14}$-$C_{22}$-Fettsäure mit der Formel: -CO-($C_xH_yZ_z$)$CH_3$ darstellt,
  wobei Z -OH oder ein Epoxidsauerstoff ist, x eine ganze Zahl von 12 bis 20 ist, y eine ganze Zahl von 20 bis 40 ist und z 0 oder eine ganze Zahl von 1 bis 4 ist,

- $R^1$ einen aliphatischen linearen oder verzweigten, gesättigten oder ungesättigten C8-C28-Rest darstellt, der gegebenenfalls mit einem Hydroxylrest substituiert ist, und
- $R^2$ H, einen Phosphat-, Sulfatrest oder einen Zucker darstellt.

**2.** Zusammensetzung nach dem vorangehenden Anspruch, in der der Mikroorganismus der Gattung *Bifidobacterium*

*species* aus *Bifidobacterium longum, Bifidobacterium bifidum, Bifidobacterium breve, Bifidobacterium animalis, Bifidobacterium lactis, Bifidobacterium infantis, Bifidobacterium adolescentis*, *Bifidobacterium pseudocatenulatum* und ihren Gemischen ausgewählt ist.

**3.** Zusammensetzung nach einem der vorangehenden Ansprüche, in der der Mikroorganismus der Gattung *Bifidobacterium species* das *Bifidobacterium longum* ist.

**4.** Zusammensetzung nach einem der vorangehenden Ansprüche, in der das Lysat mit mindestens 0,001 % (als Trockengewicht ausgedrückt), insbesondere mit 0,01 bis 20 % und spezieller mit 0,1 bis 15 % Trockengewicht des Wirkstoffs in Bezug auf das Gesamtgewicht der Zusammensetzung eingesetzt wird.

**5.** Zusammensetzung nach Anspruch 1, in der R und $R^2$ jeweils ein Wasserstoffatom darstellen und $R^1$ einen linearen und gesättigten Alkylrest darstellt.

**6.** Zusammensetzung nach einem der vorangehenden Ansprüche, in der das Phytosphingosin-Salicylat-Derivat

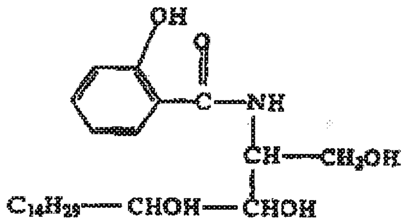

(V)

oder eines seiner Salze ist.

**7.** Zusammensetzung nach einem der vorangehenden Ansprüche, in der das Phytosphingosin-Salicylat-Derivat mit mindestens 0,0001 % (als Trockengewicht ausgedrückt), insbesondere mit 0,001 bis 20 % und spezieller mit 0,05 bis 2 % Trockengewicht des Wirkstoffs in Bezug auf das Gesamtgewicht der Zusammensetzung eingesetzt wird.

**8.** Zusammensetzung nach einem der vorangehenden Ansprüche, wobei die Zusammensetzung für eine Verabreichung über den topischen Weg bestimmt ist.

**9.** Kosmetische Verwendung eines Verbunds mit mindestens einem Lysat mindestens eines Mikroorganismus der Gattung *Bifidobacterium species* und mindestens einem Phytosphingosin-Salicylat-Derivat, wie in den Ansprüchen 1, 5 oder 6 definiert, zum Stimulieren der Expression mindestens eines Gens, das aus den Genen KRTB6, KRT10 und Involukrin ausgewählt ist, bei einem Individuum, das einer Hautalterung unterlegt.

**10.** Kosmetische Verwendung eines Verbunds mit mindestens einem Lysat mindestens eines Mikroorganismus der Gattung *Bifidobacterium species* und mindestens einem Phytosphingosin-Salicylat-Derivat, wie in den Ansprüchen 1, 5 oder 6 definiert, zum Verstärken der Reparatur- und Regenerationsfähigkeit eines Epithels, insbesondere einer besonders gealterten Epidermis.

**11.** Verwendung nach einem der Ansprüche 9 und 10, bei der das Lysat wie in den Ansprüchen 2 bis 4 definiert ist.

**12.** Verfahren zur kosmetischen Behandlung, umfassend zumindest die Verabreichung mindestens eines Verbunds mit mindestens einem Lysat mindestens eines Mikroorganismus der Gattung *Bifidobacterium species* und mindestens einem Phytosphingosin-Salicylat-Derivat, wie in den Ansprüchen 1, 5 oder 6 definiert, an ein Individuum, das einer Hautalterung unterliegt.

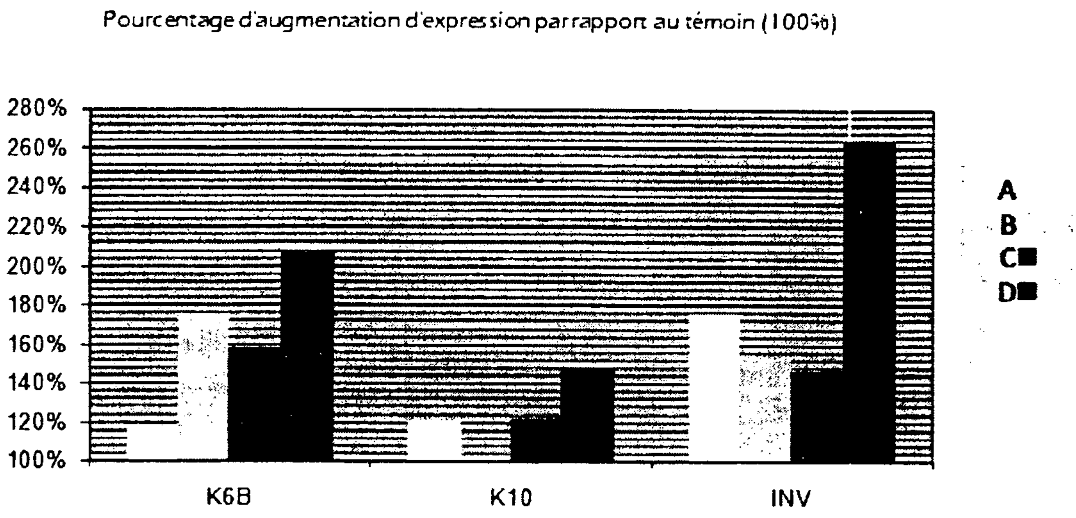
Pourcentage d'augmentation d'expression par rapport au témoin (100%)
280%
260%
240%
220%
200%
180%
160%
140%
120%
100%
K6B
K10
INV
A
B
C
D

FIGURE 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0043128 A
- WO 0228402 A
- EP 1609463 A
- EP 1642570 A
- EP 1731137 A
- FR 2876029
- FR 2006050768 W
- US 5326565 A
- EP 0919226 A
- US 5882665 A
- US 4464362 A
- EP 919226 A
- WO 2006000992 A
- WO 2006037922 A


### Littérature non-brevet citée dans la description


- **G Paragh et al.** *Exp Dermatol.,* Décembre 2008, vol. 17 (12), 1004-16
- *Joint FAO/WHO Expert Consultation on Evaluation of Health and Nutritional Properties of Probiotic in Food Including Powder Milk with Live Lactic Acid Bacteria,* 06 Octobre 2001